(19) Europäisches Patentamt
European Patent Office
Office européen des brevets

(11) Publication number: 0 262 903 A2

(12) # EUROPEAN PATENT APPLICATION

(21) Application number: 87308570.8

(22) Date of filing: 28.09.87

(51) Int. Cl.[4]: C 07 H 17/08
A 61 K 31/70

(30) Priority: 29.09.86 US 912880

(43) Date of publication of application:
06.04.88 Bulletin 88/14

(84) Designated Contracting States:
AT BE CH DE ES FR GB GR IT LI NL SE

(71) Applicant: ELI LILLY AND COMPANY
Lilly Corporate Center
Indianapolis Indiana 46285 (US)

(72) Inventor: Kirst, Herbert Andrew
1819 Madison Village Drive, Apt. B-6
Indianapolis Indiana 46227 (US)

Leeds, James Patrick
517 Stony Creek Circle
Noblesville Indiana 46060 (US)

(74) Representative: Tapping, Kenneth George et al
Erl Wood Manor
Windlesham Surrey, GU20 6PH (GB)

(54) **New acyl derivatives of 20-modified tylosin and desmycosin compounds.**

(57) New 4‴-O-acyl-20-modified tylosin and 4″-O-acyl-20-modified-desmycosin and 4′-deoxydesmycosin derivatives of formula 1 have significant oral antibacterial activity. Compositions containing and methods of using these compounds are also provided.

EP 0 262 903 A2

**Description**

NEW ACYL DERIVATIVES OF 20-MODIFIED TYLOSIN AND DESMYCOSIN COMPOUNDS

This invention relates to new antibiotics. In particular, this invention relates to a group of derivatives of the macrolide antibiotics tylosin and desmycosin and to the acid addition salts of these derivatives. This invention also relates to methods of treating certain infections with, methods of promoting growth in animals with, and pharmaceutical compositions comprising the specified derivatives and their pharmaceutically acceptable acid addition salts.

New, improved antibiotics are continually in demand. In addition to antibiotics which are useful for treating human diseases, improved antibiotics are also needed in the veterinary field. Increased potency, expanded spectrum of bacterial inhibition, increased in vivo efficacy, and improved pharmaceutical properties (such as greater oral absorption, higher blood or tissue concentrations, longer body half-life, and more advantageous rate or route of excretion and rate or pattern of metabolism) continue to be goals for improved antibiotics.

A large number of derivatives of tylosin and related macrolides have been made. Unfortunately, many of these derivatives have been either less effective or no better than the parent compounds. One group of superior derivatives was obtained by reductive or protective chemical modification of the C-20 aldehyde group.

We have now discovered another series of derivatives with significant antibiotic activity and oral efficacy. In this series of compounds, the 4'''- hydroxyl group of tylosin or the analogous 4''-hydroxyl group of desmycosin has been acylated.

According to the present invention, there is provided tylosin and desmycosin derivatives having formula 1:

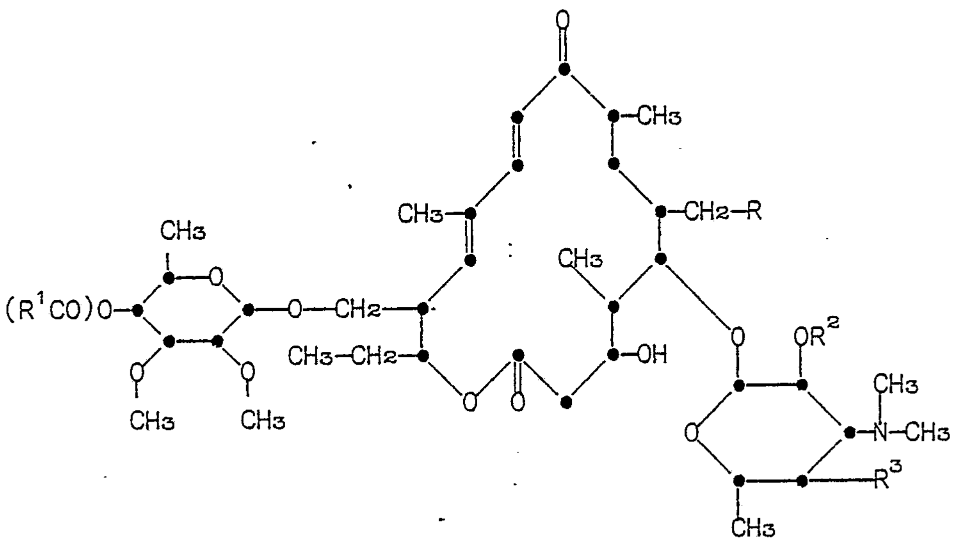

1

wherein

R is $CH_2Z$, 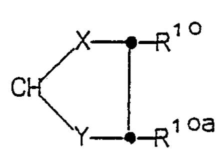 or 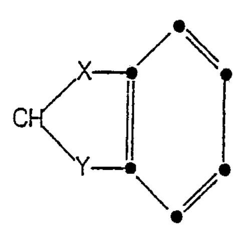 ;

Z is $OR^4$, $SR^5$, Cl, F, $N_3$ or $NR^6R^7$;

X and Y independently represent O, S, N-$CH_3$ N-phenyl or N-benzyl;

$R^1$ is hydrogen, phenyl, $R^{11}$-substituted phenyl, $C_1$-$C_4$-alkyl or $C_1$-$C_4$-alkyl having one to three halo, $C_1$-$C_3$-alkoxy, hydroxyl, acetoxy, protected-amino, phenyl, $R^{11}$-substituted-phenyl, phenoxy, $R^{11}$-substituted-phenoxy, $NR^{12}R^{13}$ or $C_3$-$C_6$-cycloalkyl substituents;

$R^2$ is hydrogen, $C_1$-$C_5$-alkanoyl, halo-substituted-$C_1$-$C_5$-alkanoyl, or benzoyl, phenylacetyl or phenylpropionyl, each of which may have an $R^{11}$ substituent on the phenyl ring;

$R^3$ is hydrogen, $OR^2$ or

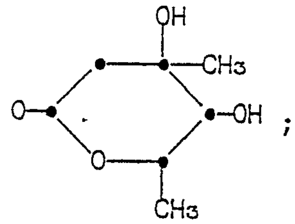

(mycarosyloxy)

$R^4$ is $C_1$-$C_4$-alkyl; $C_1$-$C_4$-alkanoyl; cyclohexyl; phenyl, benzyl, phenethyl or phenoxyethyl, each of which may have an $R^{11}$ substituent on the ring; or a heteroaryl group selected from pyridinyl, pyrimidinyl, pyridazinyl or pyrazinyl;

$R^5$ is $C_1$-$C_4$-alkyl; cyclohexyl; phenyl, benzyl or phenethyl, each of which may have an $R^{11}$ substituent on the phenyl ring; or a heteroaryl group selected from pyridinyl, tetrazolyl, oxazolyl or thiazolyl;

$R^6$ and $R^7$ independently are $C_1$-$C_8$-alkyl or a group of the formula:

$(CH_2)_n(Cyc)$

where n is 0, 1 or 2, and Cyc is $C_3$-$C_8$-cycloalkyl, phenyl or $R^{11}$-substituted phenyl; or taken together with the adjacent nitrogen atom form a saturated or unsaturated heterocyclic monocyclic ring containing from 5 to 16 ring atoms or a bicyclic or tricyclic ring system containing from 8 to 20 ring atoms wherein one or more of the ring atoms may be substituted by $C_1$-$C_4$-alkyl, $C_1$-$C_4$-alkoxy, $C_1$-$C_4$-alkoxycarbonyl, hydroxy, $C_1$-$C_4$-alkanoyloxy, halo, $NR^8R^9$, phenyl or $R^{11}$-substituted phenyl;

$R^8$ and $R^9$ independently are $C_1$-$C_4$-alkyl or $(CH_2)_n(Cyc)$; or taken together with the adjacent nitrogen atom form a saturated heterocyclic monocyclic ring containing from 5 to 8 ring atoms;

$R^{10}$ and $R^{10a}$ independently are hydrogen, methyl, phenyl, methoxycarbonyl, ethoxycarbonyl or phenoxycarbonyl; and

$R^{11}$ is halo, $C_1$-$C_3$-alkyl, $C_1$-$C_3$-alkoxy, nitro or hydroxy; and

$R^{12}$ and $R^{13}$ independently are hydrogen, $C_1$-$C_4$-alkyl, $(CH_2)_n$ (Cyc) or $R^{11}$-substituted-$(CH_2)_n(Cyc)$; or taken together with the adjacent nitrogen atom form a saturated heterocyclic monocyclic ring containing from 5 to 8 ring atoms or an $R^{11}$-substituted saturated heterocyclic monocyclic ring containing 5 to 8 ring atoms;

and the acid addition salts of these compounds.

The compounds of this invention are useful as antibiotics and/or as intermediates to antibiotics. This invention also relates to pharmaceutical compositions comprising these compounds and to methods of treatment wherein these compounds or compositions are administered to obtain an antibiotic effect or to enhance growth promotion in animals.

Although no stereochemical assignments are indicated in the structures given herein, the stereochemistry is identical to that of the antibiotics from which the compounds are prepared, i.e. tylosin and desmycosin.

The term "alkyl" means a hydrocarbon group containing the specified number of carbon atoms. Such groups can be straight, branched or cyclic and can be saturated or unsaturated. The term "cycloalkyl" means a cyclic hydrocarbon group containing the specified number of carbon atoms; such groups can also be saturated or unsaturated. By unsaturated is meant a hydrocarbon group containing double or triple bonds.

The term "alkanoyl" refers to an acyl moiety derived from a carboxylic acid containing the specified number of carbon atoms. When halo-substituted, the alkanoyl group bears from one to three halo substituents. Acetyl, chloroacetyl, trichloroacetyl, trifluoroacetyl, propionyl, n-butyryl, isobutyryl, n-valeryl and isovaleryl are examples of alkanoyl or halo-substituted alkanoyl groups.

The term "protected-amino" means that the amino group is substituted by a suitable protecting group. Such a group must be compatible with the other functional groups in the macrolide such that it is readily removed under conditions which leave the rest of the macrolide intact. Appropriate amino-protecting groups are well known (see, for example, T.W. Greene, "Protective Groups in Organic Synthesis," John Wiley and Sons, New York, 1981, Chapter 7). Examples of suitable amino-protecting groups include carbamates such as t-butyl carbamate (BOC group), benzyl carbamate (CBZ group), methyl carbamate and substituted ethyl carbamate such as 2,2,2-trichloroethyl carbamate; amides such as formamide; imides such as phthalimide; N-aralkyl derivatives such as N-benzyl derivatives; and amino acetal derivatives such as N-methoxymethyl derivatives. One especially suitable amino-protecting group is the BOC group.

The term halo refers to a member of the group consisting of Cl, Br, I and F.

When Z is $NR^6R^7$ and the $NR^6R^7$ group is cyclic and unsaturated, representative groups are 1,2,3,6-tetrahydropyridin-1-yl; 1,2,3,4-tetrahydroquinolin-1-yl; 1,2,3,4-tetrahydroisoquinolin-2-yl; indol-1-yl; isoindol-2-yl; indolin-1-yl; isoindolin-2-yl; 2,3,4,5-tetrahydro-1H-1-benzazepin-1-yl; 2,3,4,5-tetrahydro-1H-2-benzazepin-2-yl; 2,3,4,5-tetrahydro-1H-3-benzazepin-3-yl; pyrrol-1-yl; 1H-azepin-1-yl; carbazol-9-yl; 9,10-dihydroacridin-10-yl; and acridin-9-one-10-yl.

When Z is $NR^6R^7$ and the $NR^6R^7$ group is a saturated monocyclic ring, representative groups include

pyrrolidin-1-yl, piperidin-1-yl, morpholin-4-yl, thiomorpholin-4-yl, piperazin-1-yl, hexahydroazepin-1-yl, octahydroazocin-1-yl, octahydro-1H-azonin-1-yl, and the like.

When Z is $NR^6R^7$ and the $NR^6R^7$ group is a saturated bicyclic or tricyclic ring, representative groups include decahydroquinolin-1-yl; decahydroisoquinolin-2-yl; decahydrocyclohepta[b]pyrrol-1-yl; decahydrocyclohepta[c]pyrrol-2-yl; decahydrocyclopent[c]azepin-2-yl; decahydrocyclopent[d]azepin-3-yl; an azabicycloheptanyl group such as 3-azabicyclo[3.2.0]heptan-3-yl; an azabicyclooctanyl group such as 6-azabicyclo[3.2.1]octan-6-yl; an azabicyclononanyl group such as 3-azabicyclo[3.2.2]nonan-3-yl; an azabicyclodecanyl group such as 4-azabicyclo[5.3.0]decan-4-yl; an azatricyclo group such as 2-azatricyclo[6.2.2.$2^{3,6}$]tetradecan-2-yl or dodecahydrocarbazol-9-yl; and a spiro-fused system such as 1-azaspiro[4.5]decan-1-yl.

Representative groups when the $NR^6R^7$ group is a ring wherein one or more of the carbon atoms are substituted include 1,3,3-trimethyl-6-azabicyclo[3.2.1]octan-6-yl; 4-piperidinopiperidin-1-yl; 3,3,5-trimethylhexahydroazepin-1-yl; 4-phenylpiperidin-1-yl; 3,5-dimethylpiperidin-1-yl; N-methylpiperazinyl; and the like.

The formula 1 derivatives are prepared from tylosin, desmycosin and 4′-deoxydesmycosin via C-20 modified derivatives of these compounds. Tylosin and desmycosin are described by R. L. Hamill et al. in U.S. Patent No. 3,178,341, issued April 13, 1965. 4′-Deoxy desmycosin is prepared via procedures outlined in J. Antibiotics 34, 1381-1384 (1981). The structures of these starting materials are shown in formulas 2-7:

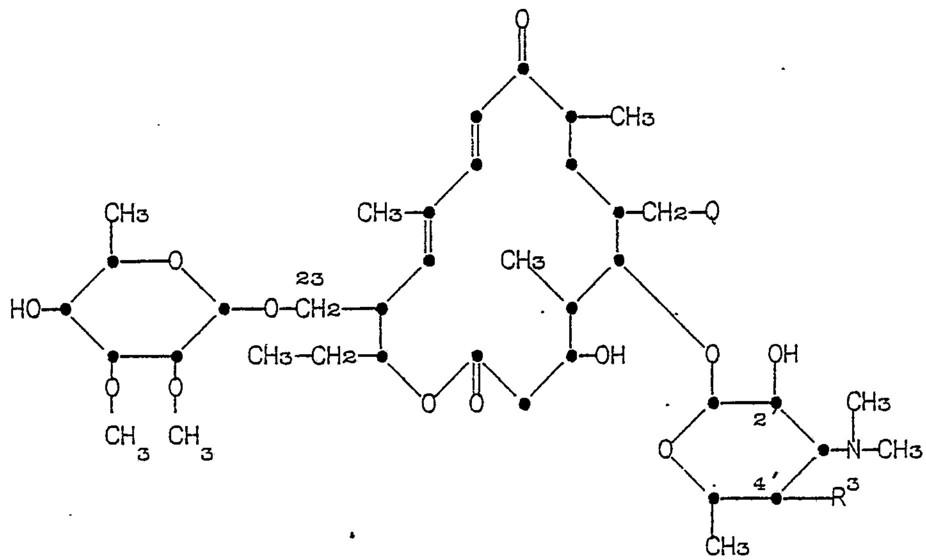

| | Q | $R^3$ |
|---|---|---|
| 2 (tylosin): | -CHO | -O-mycarosyl |
| 3 (desmycosin): | -CHO | -OH |
| 4 (4'-deoxydesmycosin) | -CHO | H |
| 5 | R | -O-mycarosyl |
| 6 | R | -OH |
| 7 | R | H |

The first step in preparing the compounds of formula 1 is to modify compounds 2-4 at the C-20 position to prepare the 5-7 compounds, using procedures in the art [see, for example, U.S. Patents 4,443,436 and 4,440,759; Matsubara et al., Chem. Pharm. Bull 30(1), 97-110 (1982); Omura et al., J. Antibiotics 37(9), 1007-1015 (1984); and European Patent 0,103,465, issued 10 September 1986]. The C-20 modified cyclic amino derivatives of the copending application are prepared by two general methods.

Method 1:

In this method, the aldehyde group of compound 2, 3 or 4 is first reduced to give the corresponding 20-dihydro compound. The C-20 hydroxyl group in this compound is then converted to a leaving group suitable

for displacement reactions by one of two methods. In one method the C-20 hydroxyl group is converted to the trifluoromethanesulfonyloxy (triflate) group, which may be further converted to another leaving group such as iodo, if desired. In the other method, which can be used with either desmycosin or 4′-deoxydesmycosin, the iodo derivative is directly formed by addition of iodine (which may be dissolved in a suitable solvent such as dimethylformamide) to a solution of the 20-dihydro derivative and triphenylphosphine under nitrogen.

The leaving group at C-20 (iodo, triflate, etc.) is then displaced by reaction with the appro priate amine in a suitable solvent, such as acetonitrile, until formation of the desired 20-modified derivative is complete.

Method 2:

In this method, the aldehyde group of compound 2, 3 or 4 is reacted directly with the corresponding amine in the presence of a suitable reducing agent in an appropriate solvent until the desired product is formed. Sodium cyanoborohydride and sodium borohydride are examples of suitable reducing agents. Formic acid can also be used as the reducing agent. Anhydrous methanol is a useful solvent for the reaction. The reaction may be carried out under a nitrogen atmosphere, but this is usually not required.

The C-20-modified derivatives of desmycosin can also be prepared by acidic hydrolysis of mycarose from the corresponding C-20-modified derivatives of tylosin. Procedures for the acidic hydrolysis of mycarose from tylosin to form desmycosin are well known.

In many cases, the C-20-modified derivative of desmycosin can be converted to the C-20-modified-4′-deoxy analog by procedures similar to those used to prepare 4′-deoxydesmycosin from desmycosin.

The second step in preparing the formula 1 compounds is to protect the hydroxyl groups at the 2′ or 2′ and 4′-positions. The 2′-mono-esters of the C-20-modified tylosin and 4′-deoxydesmycosin derivatives and the 2′,4′-diesters of the C-20-modified desmycosin derivatives are prepared by esterifying the appropriate C-20-modified derivative on the appropriate hydroxyl groups by treatment with acylating agents, using standard methods well exemplified in the art (see, for example, U.S. Patent 4,443,436).

The next step in preparing the formula 1 compounds is to esterify the hydroxyl group on the mycinose moiety; this hydroxyl group is at the 4‴-position in tylosin, but is at the 4″-position in desmycosin and 4′-deoxydesmycosin. With the appropriate hydroxyl groups at 2′ and 4′ protected, the hydroxyl group on mycinose becomes the most reactive hydroxyl group remaining in these highly functionalized molecules. The acylating agents and methods of esterification are standard ones which are exemplified in the art, such as acyl chlorides or anhydrides in the presence of bases such as tertiary amines (e.g. pyridine, collidine, triethylamine, etc.).

In the final step, selective deesterification of the 2′ and/or 4′-positions using standard methanolysis conditions provides the respective 4″-or 4‴-monoacylated compounds (again, see U.S. 4,443,436 for suitable conditions).

An alternate approach to the synthesis of (alkylamino)acyl derivatives utilizes 2′ and/or 4′-acyl-4″- or 4‴-haloacyl derivatives as intermediates. The halo group is then displaced by the alkylamino group to give the 2′ and/or 4′-acyl-4″- or 4‴-($R^{12}R^{13}N$)acyl derivatives. Finally, these compounds are selectively deesterified at the 2′ and/or 4′-positions as described supra to give the desired 4″- or 4‴-($R^{12}R^{13}N$)acyl derivatives. In each of these steps, standard procedures for displacing the halo group and for selectively de-esterifying are used.

Another approach to the synthesis of (primary or secondary amino)acyl derivatives utilizes 2′ and/or 4′-acyl-4″ or 4‴-protected-aminoacyl derivatives as intermediates. These compounds are converted to the final products by removal of the amino-protecting groups on the 4″ or 4‴ acyl moiety and by selective deesterification of the 2′ and/or 4′- positions to give the 4″-or 4‴-aminoacyl derivatives. Procedures well recognized in the art for removing amino-protecting groups and for selectively de-esterifying are used.

The formula 1 compounds can form acid addition salts. These salts are also useful as antibiotics and are a part of this invention. In another aspect, the salts are useful as intermediates, for example, for separating and purifying the derivatives. In addition, the salts have an improved solubility in water.

Representative suitable salts include those salts formed by standard reactions with both organic and inorganic acids such as, for example, sulfuric, hydrochloric, phosphoric, acetic, succinic, citric, lactic, maleic, fumaric, palmitic, cholic, pamoic, mucic, D-glutamic, d-camphoric, glutaric, glycolic, phthalic, tartaric, formic, lauric, stearic, salicylic, methane sulfonic, benzenesulfonic, sorbic, picric, benzoic, cinnamic, and like acids.

Pharmaceutically acceptable acid addition salts are an especially preferred group of salts of this invention.

Illustrative formula 1 compounds are listed in Table I.

Table I

Illustrative Formula 1 Derivatives

| Compound No. | R | ($R^1CO$) Group | $R^2$ | $R^3$ |
|---|---|---|---|---|
| 1 | $CH_2$-O-phenyl | acetyl | acetyl | acetoxy |
| 2 | " | " | H | hydroxy |
| 3 | " | phenylacetyl | acetyl | acetoxy |
| 4 | " | " | H | hydroxy |
| 5 | " | N-(t-BOC)phenylglycyl | acetyl | acetoxy |
| 6 | " | " | H | hydroxy |
| 7 | " | phenylglycyl | H | " |
| 8 | " | chloroacetyl | acetyl | acetoxy |
| 9 | " | phenoxyacetyl | H | hydroxy |
| 10 | " | (N-benzyl)aminoacetyl | acetyl | acetoxy |
| 11 | " | " | H | hydroxy |
| 12 | " | (pyrrolidin-1-yl)acetyl | acetyl | acetoxy |
| 13 | " | " | H | hydroxy |
| 14 | " | (4-methylpiperazin-1-yl)acetyl | acetyl | acetoxy |

Table I continued

Illustrative Formula 1 Derivatives

| Compound No. | R | ($R^1CO$) Group | $R^2$ | $R^3$ |
|---|---|---|---|---|
| 15 | $CH_2$-O-phenyl | (4-methylpiperazin--1-yl)acetyl | H | hydroxy |
| 16 | $CH_2N(Et)$(2-Me-butyl) | acetyl | acetyl | acetoxy |
| 17 | " | " | H | hydroxy |
| 18 | $CH_2$(3,5-dimethyl-piperidinyl) | " | acetyl | acetoxy |
| 19 | " | " | H | hydroxy |
| 20 | $CH_2$-O-phenyl | " | acetyl | H |
| 21 | " | " | H | H |
| 22 | " | phenylacetyl | acetyl | mycarosyloxy |
| 23 | " | " | H | " |
| 24 | $CH_2N(Pr)_2$ | acetyl | acetyl | " |
| 25 | " | " | H | " |
| 26 | $CH_2$(3,5-dimethyl-piperidinyl) | " | acetyl | H |
| 27 | " | " | H | H |
| 28 | $CH_2$-O-phenyl | formyl | acetyl | acetoxy |

The derivatives of this invention inhibit the growth of pathogenic bacteria, especially Gram-positive bacteria, Mycoplasma species and Gram-negative bacteria such as Pasteurella species. The derivatives have

unexpectedly useful in vivo activity. Particularly unexpected is the ability of these derivatives to treat infections successfully when administered orally.

The minimal inhibitory concentrations (MIC's) at which illustrative compounds inhibit certain bacteria are given in Tables II and III. The MIC's in Table II were determined by standard agar-dilution assays. The MIC's in Table III were obtained using conventional broth-dilution microtiter tests.

Table II

Antibiotic Activity of Formula I Compounds[a]

| Test Organism | Test Compound[b] 1 | 2 | 4 | 6 | 7 | 8 | 11 | 13 | 15 | 17 | 19 | 28 |
|---|---|---|---|---|---|---|---|---|---|---|---|---|
| Staphylococcus aureus X1.1 | 4 | 2 | 2 | 8 | 1 | 2 | 1 | 0.5 | 4 | 8 | 2 | 2 |
| Staphylococcus aureus V41[c] | 2 | 2 | 2 | 8 | 2 | 2 | 2 | 1 | 4 | 8 | 1 | 2 |
| Staphylococcus aureus X400[d] | 4 | 2 | 2 | 8 | 1 | 2 | 2 | 1 | 4 | 4 | 1 | 1 |
| Staphylococcus aureus S13E | 4 | 2 | 2 | 16 | 1 | 2 | 2 | 1 | 4 | 8 | 1 | 1 |
| Staphylococcus epidermidis EPI1 | 4 | 2 | 2 | 16 | 2 | 2 | 2 | 1 | 8 | 8 | 1 | 1 |
| Staphylococcus epidermidis 222 | 1 | 1 | 1 | 8 | 1 | 2 | 2 | 0.5 | 4 | 4 | 1 | 1 |
| Streptococcus pyogenes C203 | 1 | 1 | 2 | 8 | 2 | 2 | 2 | 1 | 2 | 4 | 0.125 | 2 |
| Streptococcus pneumoniae Park I | 8 | 2 | 4 | 16 | 2 | 8 | 2 | 2 | 1 | 4 | 0.125 | 4 |
| Streptococcus faecalis X66 | 64 | 16 | 16 | - | 16 | 32 | 8 | 16 | 16 | 64 | 8 | 8 |
| Streptococcus faecalis 2041 | 128 | 32 | 64 | - | 32 | 128 | 8 | 16 | 16 | 64 | 8 | 16 |
| Haemophilus influenzae C.L.[e] | -[g] | 128 | 128 | - | 128 | - | 128 | 32 | 128 | 64 | 32 | 64 |
| Haemophilus influenzae 76[f] | - | 128 | 128 | - | 64 | - | 64 | 32 | 128 | 128 | 64 | NT |
| Escherichia coli EC14 | - | - | - | - | - | - | - | - | - | - | - | - |
| Klebsiella pneumoniae X68 | - | - | - | - | - | - | - | - | - | - | - | - |
| Pseudomonas aeruginosa X239 | - | - | - | - | - | - | - | - | - | - | - | - |

[a]MIC in mcg/ml; [b]Compound numbers from Table I; [c]Penicillin-resistant strain; [d]Methicillin-resistant strain; [e]Ampicillin-sensitive strain; [f]Ampicillin-resistant strain; [g]Not active at 128 mcg/ml, the highest level tested

Table III

Antibiotic Activity of Formula 1 Compounds

| Test Organism | Test Compound[b] 2 | 13 |
|---|---|---|
| *Staphylococcus aureus* | 1.56 | 3.12 |
| *Streptococcus* sp. 19F | 12.5 | 6.25 |
| *Pasteurella multocida* 17E[c] | 50 | 50 |
| *Pasteurella multocida* 60A[d] | -[e] | 50 |
| *Pasteurella multocida* 40G | 50 | 25 |
| *Pasteurella multocida* 22A | - | 50 |
| *Pasteurella multocida* 68C | 25 | 25 |
| *Pasteurella hemolytica* 23C | 50 | 50 |
| *Pasteurella hemolytica* 41D | - | - |
| *Pasteurella hemolytica* 22C | - | - |
| *Bordetella bronchiseptica* | - | - |
| *Mycoplasma gallisepticum* 29C | 0.78 | 1.56 |
| *Mycoplasma gallisepticum* 15E | 50 | 50 |
| *Mycoplasma gallisepticum* 36H | 50 | 50 |
| *Mycoplasma synoviae* 40A | 3.12 | NT[f] |
| *Mycoplasma hyorhinis* 29E | 50 | NT |
| *Mycoplasma hyopneumoniae* S5972 | 25 | - |

[a]MIC in mcg/mL

[b]Compound numbers from Table I

[c]Bovine isolate

[d]Avian isolate

[e]not active at 50 mcg/mL

[f]NT = not tested

The formula 1 compounds have shown *in vivo* antimicrobial activity against experimentally-induced infections in laboratory animals. When two doses of test compound were administered to mice experimentally infected with *S. pyogenes* C203, the activity observed was measured as an $ED_{50}$ value [effective dose in mg/kg to protect 50% of the test animals: see Warren Wick, et 5 al., *J. Bacteriol.* 81, 233-235 (1961)]. $ED_{50}$ values observed for illustrative compounds are given in Table IV.

## Table IV

ED$_{50}$ Values of Formula 1 Compounds vs. *Streptococcus pyogenes* C203 in Mice[a]

| Test Compound[b] | Subcutaneous | Oral |
|---|---|---|
| 2 | 4.46 | 10.9 |
| 4 | >10 | 14.2 |
| 7 | >10. | 21 |
| 11 | 5.7 | 14.4 |
| 13 | 10 | 24.0 |
| 15 | >10 | 29.0 |

[a]mg/kg x 2; doses given 1 and 4 hours post-infection

[b]Compound numbers from Table I.

This invention also relates to methods of controlling infections caused by bacterial and mycoplasmal species. In carrying out the methods of this invention, an effective amount of a formula 1 compound is administered parenterally or orally to an infected or susceptible warm-blooded animal.

The dose which is effective to control the infection will vary with the severity of the infection and the age, weight, and condition of the animal. The total dose required for protection parenterally will generally, however, be in the range of from about 0.1 to about 100 mg/kg and preferably will be in the range of from about 0.1 to about 30 mg/kg. The dose required for oral administration will generally be in the range of from about 1 to about 300 mg/kg and preferably will be in the range of from about 1 to about 100 mg/kg. Suitable dosage regimens can be constructed.

Often the most practical way to administer the compounds is by formulation into the feed supply or drinking water. A variety of feeds, including the common dry feeds, liquid feeds, and pelleted feeds, may be used.

In another aspect, this invention relates to compositions useful for the control of infections caused by bacteria and *Mycoplasma* species. These compositions comprise a formula 1 compound together with a suitable vehicle. Compositions may be formulated for parenteral or oral administration by methods recognized in the pharmaceutical art.

The methods of formulating drugs into animal feeds are well-known. A preferred method is to make a concentrated-drug premix which in turn is used to prepare medicated feeds. Typical premixes may contain from about 1 to about 200 grams of drug per pound of premix. Premixes may be either liquid or solid preparations.

The final formulation of feeds for animals or poultry will depend upon the amount of drug to be administered. The common methods of formulating, mixing, and pelleting feeds may be used to prepare feeds containing a formula 1 compound.

Effective injectable compositions containing these compounds may be in either suspension or solution form. In the preparation of suitable formulations it will be recognized that, in general, the water solubility of the acid addition salts is greater than that of the free bases. Similarly, the bases are more soluble in dilute acids or in acidic solutions than in neutral or basic solutions.

In the solution form the compound is dissolved in a physiologically acceptable vehicle. Such vehicles comprise a suitable solvent, preservatives such as benzyl alcohol, if needed, and buffers. Useful solvents include, for example, water and aqueous alcohols, glycols, and carbonate esters such as diethyl carbonate.

Injectable suspension compositions require a liquid suspending medium, with or without adjuvants, as a vehicle. The suspending medium can be, for example, aqueous polyvinylpyrrolidone, inert oils such as vegetable oils or highly refined mineral oils, or aqueous carboxymethylcellulose.

Suitable physiologically acceptable adjuvants are necessary to keep the compound suspended in suspension compositions. The adjuvants may be chosen from among thickeners such as carboxymethylcellulose, polyvinylpyrrolidone, gelatin, and the alginates. Many surfactants are also useful as suspending agents. Lecithin, alkylphenol polyethylene oxide adducts, naphthalenesulfonates, alkylbenzenesulfonates, and the polyoxyethylene sorbitan esters are useful suspending agents.

Many substances which affect the hydrophilicity, density, and surface tension of the liquid suspending medium can assist in making injectable suspensions in individual cases. For example, silicone antifoams, sorbitol, and sugars can be useful suspending agents.

In order to illustrate more fully the operation of this invention, the following examples are provided. In these examples the abbreviation "20-DH" is used for the term "20-dihydro".

Preparation 1

A. Purification of cis-3,5-Dimethylpiperidine

A solution containing 3,5-dimethylpiperidine (40 mL, a 4:1 mixture of cis:trans isomers), triethylamine (42 mL) and dichloromethane (250 mL) was stirred while o-chlorobenzoyl chloride (38.2 mL) was added dropwise at a rate sufficient to maintain gentle reflux. At the completion of the addition, the solution was stirred for another half hour; then ten percent aqueous ammonium chloride solution (200 mL) and sufficient conc. HCl to make the aqueous layer acidic were added. The organic layer was separated, washed with brine, dried and concentrated. The solid obtained was recrystallized twice from $CH_2Cl_2$ and hexane to give essentially pure cis-amide, mp 99-103°C.

The amide (17 g) was dissolved in ethylene glycol. Potassium hydroxide pellets (11 g) were added, and the solution was heated to reflux. After one hour, the flask was arranged for downward distillation, and the fraction boiling between 100°C and 195°C was collected. This material was partitioned between water and ether, dried and concentrated to afford pure cis-3,5-dimethylpiperidine.

B. Alternate Purification of cis-3,5-Dimethylpiperidine

A 1-liter, 3-neck flask was charged with 48 mL (0.36 mol) of commercial grade 3,5-dimethylpiperidine (ca. 80-85% cis) and 600 mL of anhydrous ether. HCl gas was bubbled through the solution with vigorous stirring until no free amine remained. The product which formed was separated by filtration and air-dried to give 47 g of the hydrochloride salt, mp 160-180°C.

The salt was suspended in acetone (600 mL) and heated to reflux for 1 hr. The reaction mixture was cooled to 50°C and filtered; the separated solid was air-dried to yield 25.6 g of product, mp 226-228°C. A portion of this material (18 g) was dissolved in water (100 mL), and the solution was adjusted to pH 10 with sodium hydroxide pellets. The free amine was extracted into diethyl ether, dried over magnesium sulfate, filtered and carefully concentrated to yield 8.5 mL of cis-3,5-dimethylpiperidine, contaminated with ≦5% of the trans isomer.

C. Preparation of cis-20-DH-20-deoxy-20-(3,5-dimethylpiperidin-1-yl)desmycosin

Desmycosin (10 g, 12.9 mmol) was dissolved in dry methanol (100 mL), and cis-3,5-dimethylpiperidine (4 g, 35 mmol) was added. After the mixture was stirred for 30 minutes at room temperature, sodium cyanoborohydride (0.8 g, 12.9 mmol) was added. The solution was stirred overnight and then was evaporated under reduced pressure. The residue was partitioned between ethyl acetate and water (150 mL each). The organic layer was then extracted sequentially with pH 6.5 phosphate buffer (100 mL) and pH 4.5 phosphate buffer (100 mL).

The latter solution was then adjusted to pH 10 with 5N sodium hydroxide, and the free amine was reextracted into ethyl acetate. The solution was dried over magnesium sulfate, filtered and concentrated to yield 6.0 g of 20-DH-20-deoxy-20-(cis-3,5-dimethylpiperidine-1-yl)desmycosin. Analysis of the product by reverse-phase HPLC detected no trans-isomer.

Preparation 2

Separation of cis- and trans-20-DH-20-deoxy-20-(3,5-dimethylpiperidin-1-yl)desmycosin

20-DH-20-deoxy-20-(3,5-dimethylpiperidin-1-yl)desmycosin was prepared using 3,5-dimethylpiperidine which was a mixture of cis:trans-isomers (4:1). The product was chromatographed over silica gel using a methanol:dichloromethane:ammonium hydroxide (49.5:49.5:1) solvent system. A single major peak was eluted. The first ten percent of this peak consisted of pure cis-isomer, and the last ten percent consisted of enriched trans-isomer (cis:trans material in a ratio of 16:84).

Example 1

2′,4′,4″-Tri-O-acetyl-20-DH-20-O-phenyl-desmycosin (compound 1)

2′,4′-Di-O-acetyl-20-O-phenyl-desmycosin (1.0 g, 1.07 mmol), prepared as described in Example 18 of U.S. Patent 4,443,436, was dissolved in $CH_2Cl_2$ (9 mL). Acetyl chloride (0.08 mL, 1.1 equiv.) and pyridine (1.0 mL) were added successively by syringe. The solution was allowed to remain at 25°C for 2 hours with the exclusion of moisture. The solution was then. diluted with saturated $NaHCO_3$ solution (50 mL), and the mixture was extracted with $CH_2Cl_2$ (4 × 25 mL), dried ($Na_2SO_4$), filtered, and evaporated to dryness. The residue was chromatographed on a Chromatotron (4-mm silica-gel plate), using ethyl acetate as the eluent, to give 0.94 g (90.0%) of the title compound as a white solid foam:

UVmax (EtOH) 279 nm (ε21988)

IR ($CHCl_3$) 1742, 1675 and 1594 $cm^{-1}$
$^1H$ NMR ($CDCl_3$) $\delta$ 4.44 (dd, 4″-H) and 2.13 (s, 3H)
FDMS m/e 975 (M).

Example 2

4″-O-Acetyl-20-DH-20-O-phenyl-desmycosin (compound 2)

A solution of 2′,4′,4″-tri-O-acetyl-20-DH-20-O-phenyl-desmycosin (0.9 g, 0.92 mmol), prepared as described in Example 1, in methanol (25 mL) was heated to ~50°C for 3 hours. The solution was evaporated to dryness to give the title compound as a white solid foam:
UVmax (EtOH) 279 nm (ε22020)
IR ($CHCl_3$) 1732, 1680 and 1594 $cm^{-1}$
$^1H$ NMR ($CDCl_3$) $\delta$ 4.44 (dd, 4″-H) and 2.13 (s, 3H)
FDMS m/e 891 (M).

Example 3

2′,4′-Di-O-acetyl-20-DH-20-O-phenyl-4″-O-(phenylacetyl)-desmycosin (compound 3)

2′,4′-Di-O-acetyl-20-DH-20-O-phenyl-desmycosin (1.0 g, 1.07 mmol) was dissolved in $CH_2Cl_2$ (9 mL). Phenylacetyl chloride (0.23 mL, 2 eq.) and pyridine (1.0 mL) were added successively by syringe. The solution was allowed to remain at 25°C for 18 hours with the exclusion of moisture. The solution was added to saturated $NaHCO_3$ solution (50 mL), and the resultant mixture was extracted with $CH_2Cl_2$ (4 × 25 mL), dried ($Na_2SO_4$), filtered, and evaporated to dryness. The residue was chromatographed on a Chromatotron (4-mm silica-gel plate), using ethyl acetate as the eluent, to give 0.63 g (55.7%) of the title compound as a pale yellow solid foam:
UVmax (EtOH) 279 nm (ε24454)
IR ($CHCl_3$) 1744, 1680, and 1594 $cm^{-1}$
$^1H$ NMR ($CDCl_3$) $\delta$ 7.44-6.80 (11H, phenyl + vinyl)
4.40 (dd, 4″-H) and 3.66 (s, 2H)
FDMS m/e 1050 (M-1).

Example 4

20-DH-20-O-Phenyl-4″-O-(phenylacetyl)-desmycosin (compound 4)

A solution of 2′,4′-di-O-acetyl-20-O-phenyl-4″-O-phenylacetyl-desmycosin (500 mg, 0.48 mmol), prepared as described in Example 3, in MeOH (15 mL) was heated to ~60°C for 3 hours. The solution was evaporated to dryness to give the title compound as a pale yellow solid foam:
UVmax (EtOH) 279 nm (ε21407)
IR ($CHCl_3$) 1735, 1680 and 1596 $cm^{-1}$
$^1H$ NMR ($CDCl_3$) $\delta$ 7.42-6.80 (11H, phenyl + vinyl)
4.45 (dd, 4″-H) and 3.66 (s, 2H)
FDMS m/e 967 (M).

Example 5

2′,4′-Di-O-acetyl-4″-O-(chloroacetyl)-20-DH-20-O-phenyl-desmycosin (compound 8)

2′,4′-Di-O-acetyl-20-O-phenyl-desmycosin (10.0 g, 10.7 mmol) was dissolved in $CH_2Cl_2$ (45 mL). The solution was cooled in an ice/water bath, and pyridine (5.0 mL) was added. Chloroacetyl chloride (1.07 mL, 1.25 eq.) was added dropwise, and the solution was stirred at 0°C for 4 hours with the exclusion of moisture. The reaction was diluted with saturated $NaHCO_3$ solution (300 mL), extracted with $CH_2Cl_2$ (3 × 100 mL), dried ($Na_2SO_4$), and evaporated to dryness. The product was isolated by chromatography on a Waters Prep 500 LC, using an 8-L gradient of hexane→ethyl acetate/hexane (3:1), to give 6.65 g (61.5%) of the title compound as a white solid foam:
UVmax (EtOH) 279 nm (ε23631)
IR ($CHCl_3$) 1743, 1678, and 1594 $cm^{-1}$
$^1H$ NMR ($CDCl_3$) $\delta$ 4.52 (dd, 4″-H) and 4.09 (s, 2H)
FDMS m/e 1009 (M).

Example 6

2′,4′-Di-O-acetyl-4″-O-(N-benzylaminoacetyl)-20-DH-20-O-phenyl-desmycosin (compound 10)

2′,4′-Di-O-acetyl-4″-O-(chloroacetyl)-20-Ophenyl-desmycosin (5.70 g, 5.6 mmol) was dissolved in acetonitrile (100 mL), and benzylamine (1.85 mL, 3 eq.) was then added. The solution was allowed to remain at room temperature with the exclusion of moisture for 5 days. The mixture was filtered, and the filtrate was evaporated to dryness to give a solid foam. The foam was dissolved in $CH_2Cl_2$ (250 mL), extracted with saturated $NaHCO_3$

solution (3 × 100 mL), dried ($Na_2SO_4$), and evaporated to dryness. The residue was chromato graphed on a silica-gel flash column, using ethyl acetate/hexane (9:1) as the eluent, to give 3.70 g (60.6%) of the title compound as a white solid foam:
UVmax (EtOH) 279 nm (ε23624)
IR ($CHCl_3$) 1744, 1678 and 1594 $cm^{-1}$
$^1H$ NMR ($CDCl_3$) δ 7.40-6.80 (11H, phenyl + vinyl), 4.52 (dd, 4″-H), 3.82 (s, 2H) and 3.46 (s, 2H)
FDMS m/e 1080 (M).

Example 7

4″-O-(N-Benzylaminoacetyl)-20-DH-20-O-phenyl-desmycosin (compound 11)

2′,4′-Di-O-acetyl-4″-O-(N-benzylaminoacetyl)-20-DH-20-O-phenyl-desmycosin (3.65 g, 3.38 mmol), prepared as described in Example 6, was dissolved in methanol (35 mL) and allowed to stand at room temperature for 24 hours. The solution was evaporated to dryness to give a solid foam. The residue was chromatographed on a silica-gel flash column, using a 1-L gradient of $CH_2Cl_2$ → $CH_2Cl_2$/MeOH (9:1) followed by 500 mL of $CH_2Cl_2$/MeOH (9:1) as the eluent, to give 1.44 g (42.8%) of the title compound as a white solid foam:
UVmax (EtOH) 279 nm (ε22724)
IR ($CHCl_3$) 1742, 1720, 1678 and 1594 $cm^{-1}$
$^1H$ NMR ($CDCl_3$) δ 7.40-6.80 (11H, phenyl + vinyl), 4.52 (dd, 4″-H), 3.83 (s, 2H) and 3.46 (s, 2H)
FDMS m/e 996 (M).

Example 8

2′,4′-Di-O-acetyl-20-DH-20-O-phenyl-4″-O-(pyrrolidin-1-yl-acetyl)-desmycosin (compound 12)

Using the procedure described in Example 6, 2′,4′-di-O-acetyl-4″-O-(chloroacetyl)-20-DH-20-O-phenyl-desmycosin (3.25 g, 3.2 mmol) was reacted with pyrrolidine (0.54 mL, 3 eq) in acetonitrile (50 mL) to give a yellow solid foam. This foam was chromatographed on a silica-gel flash column using a 1-L gradient of EtOAc → EtOAc/MeOH (9:1) as the eluent to give 2.55 g (75.8%) of the title compound as a tan solid foam:
UVmax (EtOH) 278 nm (ε23609)
IR ($CHCl_3$) 1744, 1678 and 1593 $cm^{-1}$
$^1H$ NMR ($CDCl_3$) δ 4.51 (dd, 4″-H), 3.39 (s, 2H), 2.66 (bm, 4H) and 1.83 (bm, 4H)
FDMS m/e 1044 (M).

Example 9

20-DH-20-O-Phenyl-4″-O-(pyrrolidin-1-ylacetyl)-desmycosin (compound 13)

2′,4′-Di-O-acetyl-20-DH-20-O-phenyl-4″-O-(pyrrolidin-1-ylacetyl)-desmycosin (2.50 g, 2.4 mmol) in MeOH (25 mL) was allowed to stand at room temperature for 2 days. The solution was evaporated to dryness to give a pale yellow foam. This foam was chromatographed on a silica-gel flash column, using a 1-L gradient of $CH_2Cl_2$ → $CH_2Cl_2$/MeOH (9:1) followed by 500 mL of $CH_2Cl_2$/MeOH (9:1) as the eluent, to give 507 mg of the title compound as a solid foam:
UVmax (EtOH) 279 nm (ε21733)
IR ($CHCl_3$) 1745, 1715, 1680 and 1585 $cm^{-1}$
$^1H$ NMR ($CDCl_3$) δ 4.51 (dd, 4″-H), 3.39 (s, 2H), 2.67 (bm, 4H) and 1.83 (bm, 4H)
FDMS m/e 960 (M).

Example 10

2′,4′-Di-O-acetyl-4″-O-[(4-methyl-piperazin-1-yl) acetyl]-20-DH-20-O-phenyl-desmycosin (compound 14)

Using the procedure described in Example 6, 2′,4′-di-O-acetyl-4″-O-(chloroacetyl)-20-DH-20-O-phenyl-desmycosin (5.60 g, 5.6 mmol) was reacted with N-methyl-piperazine (1.23 mL, 2 eq) in acetonitrile (100 mL) to give a yellow solid foam. This foam was chromatographed on a silica-gel flash column, using a 1-L gradient of EtOAc → EtOAc/MeOH (1:1) followed by 500 mL of EtOAc/MeOH (1:1) as the eluent, to give 3.85 g (64.6%) of the title compound as a light yellow solid foam:
UVmax (EtOH) 279 nm (ε23561)
IR ($CHCl_3$) 1741, 1680 and 1594 $cm^{-1}$
$^1H$ NMR ($CDCl_3$) δ 4.50 (dd, 4″-H), 3.25 (s, 2H), 2.63 (bm, 4H), 2.50 (bm, 4H) and 2.30 (s, 3H)
FDMS m/e 1071 (M-2).

Example 11

4''-O-[(4-Methyl-piperazin-1-yl)acetyl]-20-DH-20-O-phenyl-desmycosin (compound 15)

2',4'-Di-O-acetyl-4''-O-[(4-methyl-piperazin-1-yl)acetyl]-20-DH-20-O-phenyl-desmycosin (3.70 g, 3.4 mmol) in MeOH (35 mL) was allowed to stand at room temperature for 18 hours. The solution was evaporated to dryness to give a pale yellow solid foam. This foam was chromatographed on a silica-gel flash column, using a 1-L gradient of $CH_2Cl_2 \rightarrow CH_2Cl_2/MeOH/NH_4OH$ (90:10:0.5) followed by 750 mL of $CH_2Cl_2/MeOH/NH_4OH$ (90:10:0.5) as the eluent, to give 1.95 g (57.2%) of the title compound as a white solid foam:
UVmax (EtOH) 279 nm (ε23288)
IR ($CHCl_3$) 1747, 1714, 1678 and 1594 $cm^{-1}$
$^1H$ NMR ($CDCl_3$) δ 4.52 (dd, 4''-H), 3.24 (s, 2H), 2.63 (bm, 4H), 2.50 (overlap with 3'-dimethylamino) and 2.30 (s, 3H)
FDMS m/e 990 (M+H).

Example 12

2',4'-Di-O-acetyl-4''-O-(N-BOC)-D-phenylglycyl-20-DH-20-O-phenyl-desmycosin (Compound 5)

2',4'-Di-O-acetyl-20-DH-20-O-phenyl-desmycosin (3.0 g, 3.22 mmol) and N-oxysuccinimide (N-BOC)-D-phenylglycinate (2.25 g, 2 eq) were dissolved in $CH_2Cl_2$ (45 mL); pyridine (5 mL) and 4-(dimethylamino)pyridine (39 mg, 0.1 eq) were added successively. The solution was allowed to stand at room temperature with the exclusion of moisture for 7 days. After 24 hours, additional (N-BOC)-D-phenylglycine active ester (1.25 g) was added to the solution. The reaction solution was added to saturated $NaHCO_3$ (150 mL), extracted with $CH_2Cl_2$ (2 × 50 mL), dried ($Na_2SO_4$), filtered and evaporated to dryness. The residue was purified by silica-gel flash chromatography, eluting with ethyl acetate/hexane (2:1) to give 1.85 g (49.3%) of the title compound as a white solid foam:
UVmax (EtOH) 279 nm (ε22018)
IR ($CHCl_3$) 1744, 1713, 1678 and 1591 $cm^{-1}$
$^1H$ NMR ($CDCl_3$) δ 7.54-6.84 (11H, phenyl + vinyl),
4.53 (dd, 4''-H), 3.54 (overlap) and 1.43 (s, 9H)
FDMS m/e 1162.(M-4).

Example 13

4''-O-(N-BOC)-D-phenylglycyl-20-DH-20-O-phenyl-desmycosin (Compound 6)

A solution of 2',4'-di-O-acetyl-4''-O-(N-BOC)-D-phenylglycyl-20-DH-20-O-phenyl-demycosin (1.50 g, 1.29 mmol) in MeOH (40 mL) was allowed to stand at room temperature for 45 hours. The solution was evaporated to dryness to give the title compound as a white solid foam:
UVmax (EtOH) 279 nm (ε22533)
IR ($CHCl_3$) 1740, 1712, 1678 and 1593 $cm^{-1}$
$^1H$ NMR ($CDCl_3$) δ 7.44-6.80 (11H, phenyl + vinyl), 4.42
(dd, 4''-H), 3.54 (overlap) and 1.43 (s, 9H)
FDMS m/e 1082 (M).

Example 14

20-DH-20-O-Phenyl-4''-O-(D-phenylglycyl)-desmycosin (Compound 7)

Trifluoroacetic acid (15 mL) was added to 4''-O-(N-t-Boc)-D-phenylglycyl-20-DH-20-O-phenyl-desmycosin (1.35 g, 1.25 mmol) at 0°C under argon. After 30 minutes, diethyl ether (300 mL) was added to precipitate 20-DH-20-O-phenyl-4''-O-(D-phenylglycyl)-desmycosin as its bistriflate salt. The precipitate was collected by filtration, washed with ether (2 × 50 mL) and hexane (50 mL) to give 683 mg (45.2%) of the title compound as a white solid:
UVmax (EtOH) 279 nm (ε21935)
IR (KBr) 1754, 1677 and 1598 $cm^{-1}$
FDMS m/e 982 (M).

Example 15

2',4',4''-Tri-O-acetyl-20-DH-20-deoxy-20-(3,5-dimethylpiperidin-1-yl)-desmycosin (Compound 18)

2',4'-Di-O-acetyl-20-DH-20-deoxy-20-(3,5-dimethylpiperidin-1-yl)-desmycosin (1.0 g, 1.05 mmol) was dissolved in $CH_2Cl_2$ (9.0 mL). Acetyl chloride (0.082 mL, 1.1 eq) and pyridine (1.0 mL) were added successively by syringe. The solution was allowed to remain at 25°C with the exclusion of moisture. After 18 hours, additional acetyl chloride (0.082 mL) was added. The solution was stirred for 2.5 hours, added to saturated $NaHCO_3$ solution (50 mL), extracted with $CH_2Cl_2$ (4 × 25 mL), dried ($Na_2SO_4$), and evaporated to give a solid foam. The residue was purified by silica-gel flash chromatography, eluting with a 1-L gradient of $CH_2Cl_2 \rightarrow CH_2Cl_2/MeOH$ (13.3:1) followed by 500 mL of $CH_2Cl_2/MeOH$ (13:3:1) to give 355 mg (34.0%) of the title compound as a white

glass:
UVmax (EtOH) 282 nm (ε21616)
IR ($CHCl_3$) 1742, 1677 and 1592 $cm^{-1}$
$^1H$ NMR ($CDCl_3$) δ 4.44 (dd, 4″-H) and 2.12 (s, 3H)
FDMS m/e 995 (M+H).

Example 16

4″-O-Acetyl-20-DH-20-deoxy-20-(3,5-dimethylpiperidin-1-yl)-desmycosin (Compound 19)

A solution of 2′,4′,4″-tri-O-acetyl-20-DH-20-deoxy-20-(3,5-dimethylpiperidin-1-yl)-desmycosin (322 mg, 0.32 mmol) in MeOH (15 mL) was allowed to stand at room temperature for 48 hours. The solution was evaporated to dryness to give the title compound as a colorless glass:
UVmax (EtOH) 283 nm (ε20484)
IR ($CHCl_3$) 1737, 1675 and 1590 $cm^{-1}$
$^1H$ NMR ($CDCl_3$) δ 4.44 (dd, 4″-H) and 2.11 (s, 3H)
FDMS m/e 911 (M+H).

Example 17

2′,4′,4″-Tri-O-acetyl-20-DH-20-deoxy-20-[N-ethyl-N-(2-methylbutyl)amino]desmycosin (Compound 16)

Using a procedure like that in Example 15, 2′,4′-di-O-acetyl-20-DH-20-deoxy-20-[N-ethyl-N-(2-methylbutyl)amino]desmycosin (959 mg, 1.0 mmol) was reacted with acetyl chloride to give a solid foam. The foam was purified by silica-gel flash chromatography, eluting with a 1-L gradient of $CH_2Cl_2$ → $CH_2Cl_2$/MeOH (9:1) followed by 500 mL of $CH_2Cl_2$/MeOH (9:1) to give 186 mg (18.6%) of the title compound as a colorless glass:
UVmax (EtOH) 281 nm (ε21368)
IR ($CHCl_3$) 1741, 1678 and 1592 $cm^{-1}$
$^1H$ NMR ($CDCl_3$) δ 4.44 (dd, 4″-H) and 2.12 (s, 3H)
FDMS m/e 997 (M).

Example 18

4″-O-Acetyl-20-DH-20-deoxy-20-[N-ethyl-N-(2-methylbutyl)amino]desmycosin (Compound 17)

A solution of 2′,4′,4″-tri-O-acetyl-20-DH-20-deoxy-20-[N-ethyl-N-(2-methylbutyl)amino]desmycosin (161 mg, 0.16 mmol) in MeOH (10 mL) was allowed to stand at room temperature for 24 hours. The solution was evaporated to dryness to give the title compound as a colorless glass:
UVmax (EtOH) 282 nm (ε20903)
IR ($CHCl_3$) 1736, 1678 and 1591 $cm^{-1}$
$^1H$ NMR ($CDCl_3$) δ 4.45 (dd, 4″-H) and 2.11 (s, 3H)
FDMS m/e 913 (M).

## Claims

1. A compound of the formula:

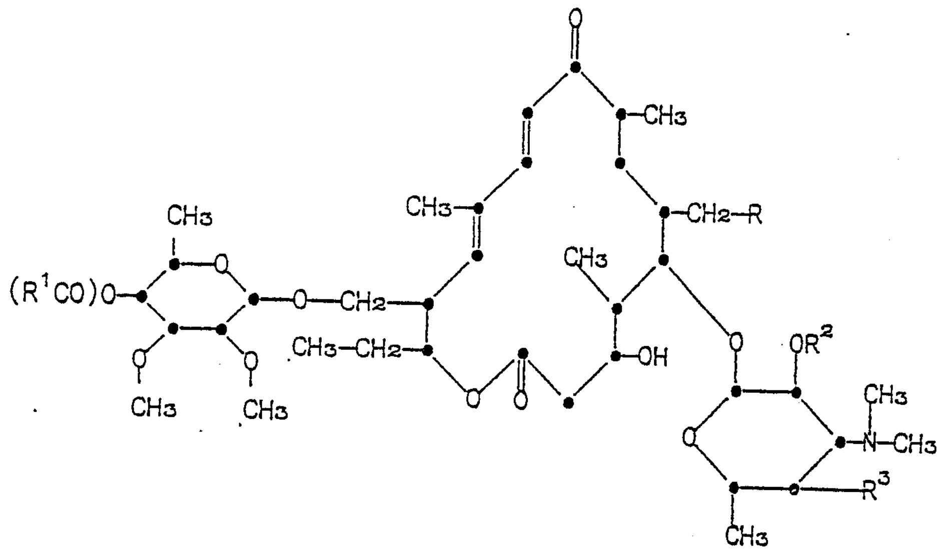

wherein

R is $CH_2Z$, 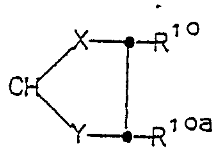 or 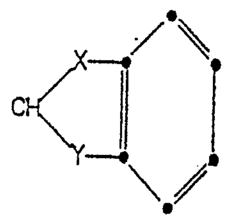 ;

Z is $OR^4$, $SR^5$, Cl, F, $N_3$ or $NR^6R^7$;

X and Y independently represent O, S, N-$CH_3$, N-phenyl or N-benzyl;

$R^1$ is hydrogen, phenyl, $R^{11}$-substituted phenyl, $C_1$-$C_4$-alkyl or $C_1$-$C_4$-alkyl having one to three halo, $C_1$-$C_3$-alkoxy, hydroxy, acetoxy, protected-amino, phenyl, $R^{11}$-substitutedphenyl, phenoxy, $R^{11}$-substituted-phenoxy, $NR^{12}R^{13}$ or $C_3$-$C_6$-cycloalkyl substituents;

$R^2$ is hydrogen, $C_1$-$C_5$-alkanoyl, halo-substituted-$C_1$-$C_5$-alkanoyl, or benzoyl, phenylacetyl or phenylpropionyl, each of which may have an $R^{11}$ substituent on the phenyl ring;

$R^3$ is hydrogen, $OR^2$ or mycarosyloxy;

$R^4$ is $C_1$-$C_4$-alkyl; $C_1$-$C_4$-alkanoyl; cyclohexyl; phenyl, benzyl, phenethyl or phenoxyethyl, each of which may have an $R^{11}$ substituent on the ring; or a heteroaryl group selected from pyridinyl, pyrimidinyl, pyridazinyl or pyrazinyl;

$R^5$ is $C_1$-$C_4$-alkyl; cyclohexyl; phenyl, benzyl or phenethyl, each of which may have an $R^{11}$ substituent on the phenyl ring; or a heteroaryl group selected from pyridinyl, tetrazolyl, oxazolyl or thiazolyl;

$R^6$ and $R^7$ independently are $C_1$-$C_8$-alkyl or a group of the formula:

$(CH_2)_n(Cyc)$

where n is 0, 1 or 2, and Cyc is $C_3$-$C_8$-cycloalkyl, phenyl or $R^{11}$-substituted phenyl; or taken together with the adjacent nitrogen atom form a saturated or unsaturated heterocyclic monocyclic ring containing from 5 to 16 ring atoms or a bicyclic or tricyclic ring system containing from 8 to 20 ring atoms wherein one or more of the ring atoms may be substituted by $C_1$-$C_4$-alkyl, $C_1$-$C_4$-alkoxy, $C_1$-$C_4$-alkoxycarbonyl, hydroxy, $C_1$-$C_4$-alkanoyloxy, halo, $NR^8R^9$ phenyl or $R^{11}$-substituted phenyl;

$R^8$ and $R^9$ independently are $C_1$-$C_4$-alkyl or $(CH_2)_n(Cyc)$; or taken together with the adjacent nitrogen atom form a saturated heterocyclic monocyclic ring containing from 5 to 8 ring atoms;

$R^{10}$ and $R^{10a}$ independently are hydrogen, methyl, phenyl, methoxycarbonyl, ethoxycarbonyl or phenoxycarbonyl; and

$R^{11}$ is halo, $C_1$-$C_3$-alkyl, $C_1$-$C_3$-alkoxy, nitro or hydroxy; and

$R^{12}$ and $R^{13}$ independently are hydrogen, $C_1$-$C_4$-alkyl, $(CH_2)_n(Cyc)$ or $R^{11}$-substituted-$(CH_2)_n(Cyc)$; or taken together with the adjacent nitrogen atom form a saturated heterocyclic monocyclic ring containing from 5 to 8 ring atoms or an $R^{11}$-substituted saturated heterocyclic monocyclic ring containing 5 to 8 ring atoms;

or an acid addition salt thereof.

2. A compound of claim 1 wherein R is $CH_2Z$.

3. A compound of claim 2 wherein Z is $OR^4$.

4. A compound of claim 2 wherein Z is $NR^6R^7$.

5. A compound of claim 3 wherein $R^4$ is phenyl, benzyl, phenethyl or phenoxyethyl, each of which may have an $R^{11}$ substituent on the ring.

6. A compound of claim 4 wherein $R^6$ and $R^7$ together with the adjacent nitrogen atom form a specified monocyclic ring which may be substituted by $C_1$-$C_4$-alkyl, $C_1$-$C_4$-alkoxy, $C_1$-$C_4$-alkoxycarbonyl, hydroxy, $C_1$-$C_4$-alkanoyloxy, halo, $NR^8R^9$, phenyl or $R^{11}$-substituted phenyl.

7. A compound of claim 6 wherein the monocyclic ring is piperidinyl or substituted piperidinyl.

8. A veterinary composition comprising a compound of any one of claims 1 to 7 and a suitable pharmaceutical vehicle.

9. A compound as claimed in any one of claims 1 to 7 for use in the therapy of microbial infections.

10. A process for preparing a compound of any one of claims 1 to 7 wherein $R^2$ is hydrogen which comprises (A) deprotecting a compound of any one of claims 1 to 7 wherein $R^2$ is other than hydrogen; or (B) acylating a compound of formula

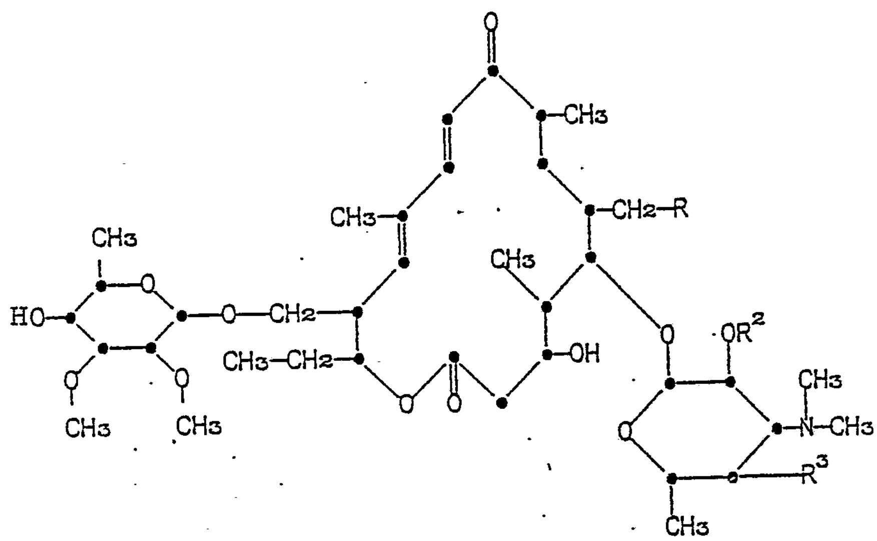

Claims for the following Contracting States: AT, GR, and ES.

1. A process for preparing a compound of the formula I:

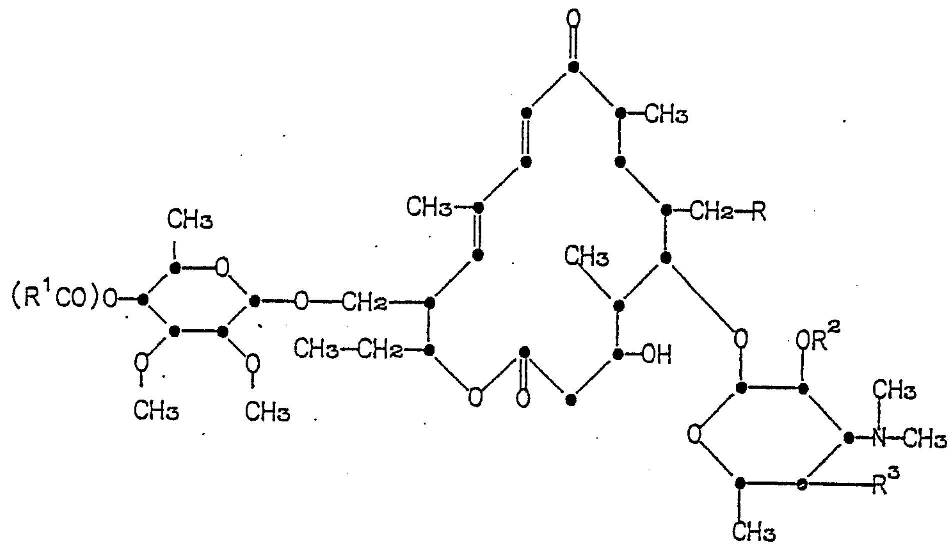

I

wherein

R is $CH_2Z$, 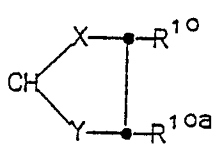 or 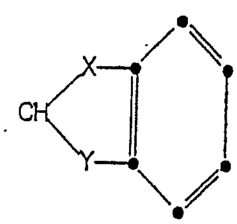 ;

Z is $OR^4$, $SR^5$, Cl, F, $N_3$ or $NR^6R^7$;

X and Y independently represent O, S, $N\text{-}CH_3$, N-phenyl or N-benzyl;

$R^1$ is hydrogen, phenyl, $R^{11}$-substituted phenyl, $C_1$-$C_4$-alkyl or $C_1$-$C_4$-alkyl having one to three halo, $C_1$-$C_3$-alkoxy, hydroxy, acetoxy, protected-amino, phenyl, $R^{11}$-substituted-phenyl, phenoxy, $R^{11}$-substituted-phenoxy, $NR^{12}R^{13}$ or $C_3$-$C_6$-cycloalkyl substituents;

$R^2$ is hydrogen, $C_1$-$C_5$-alkanoyl, halo-substituted-$C_1$-$C_5$-alkanoyl, or benzoyl, phenylacetyl or phenylpropionyl, each of which may have an $R^{11}$ substituent on the phenyl ring;

$R^3$ is hydrogen, $OR^2$ or mycarosyloxy;

$R^4$ is $C_1$-$C_4$-alkyl; $C_1$-$C_4$-alkanoyl; cyclohexyl; phenyl, benzyl, phenethyl or phenoxyethyl, each of which may have an $R^{11}$ substituent on the ring; or a heteroaryl group selected from pyridinyl, pyrimidinyl, pyridazinyl or pyrazinyl;

$R^5$ is $C_1$-$C_4$-alkyl; cyclohexyl; phenyl, benzyl or phenethyl, each of which may have an $R^{11}$ substituent on the phenyl ring; or a heteroaryl group selected from pyridinyl, tetrazolyl, oxazolyl or thiazolyl;

$R^6$ and $R^7$ independently are $C_1$-$C_8$-alkyl or a group of the formula:

$(CH_2)_n(Cyc)$

where n is 0, 1 or 2, and Cyc is $C_3$-$C_8$-cycloalkyl, phenyl or $R^{11}$-substituted phenyl; or taken together with the adjacent nitrogen atom form a saturated or unsaturated heterocyclic monocyclic ring containing from 5 to 16 ring atoms or a bicyclic or tricyclic ring system containing from 8 to 20 ring atoms wherein one or more of the ring atoms may be substituted by $C_1$-$C_4$-alkyl, $C_1$-$C_4$-alkoxy, $C_1$-$C_4$-alkoxycarbonyl, hydroxy, $C_1$-$C_4$-alkanoyloxy, halo, $NR^8R^9$ phenyl or $R^{11}$-substituted phenyl;

$R^8$ and $R^9$ independently are $C_1$-$C_4$-alkyl or $(CH_2)_n(Cyc)$; or taken together with the adjacent nitrogen atom form a saturated heterocyclic monocyclic ring containing from 5 to 8 ring atoms;

$R^{10}$ and $R^{10a}$ independently are hydrogen, methyl, phenyl, methoxycarbonyl, ethoxycarbonyl or phenoxycarbonyl; and

$R^{11}$ is halo, $C_1$-$C_3$-alkyl, $C_1$-$C_3$-alkoxy, nitro or hydroxy; and

$R^{12}$ and $R^{13}$ independently are hydrogen, $C_1$-$C_4$-alkyl, $(CH_2)_n(Cyc)$ or $R^{11}$-substituted-$(CH_2)_n(Cyc)$; or taken together with the adjacent nitrogen atom form a saturated heterocyclic monocyclic ring containing from 5 to 8 ring atoms or an $R^{11}$-substituted saturated heterocyclic monocyclic ring containing 5 to 8 ring atoms;

or an acid addition salt thereof; which comprises (A) deprotecting a compound of formula I wherein $R^2$

is other than hydrogen so as to prepare a compound of formula I wherein $R^2$ is hydrogen; or
(B) acylating a compound of formula

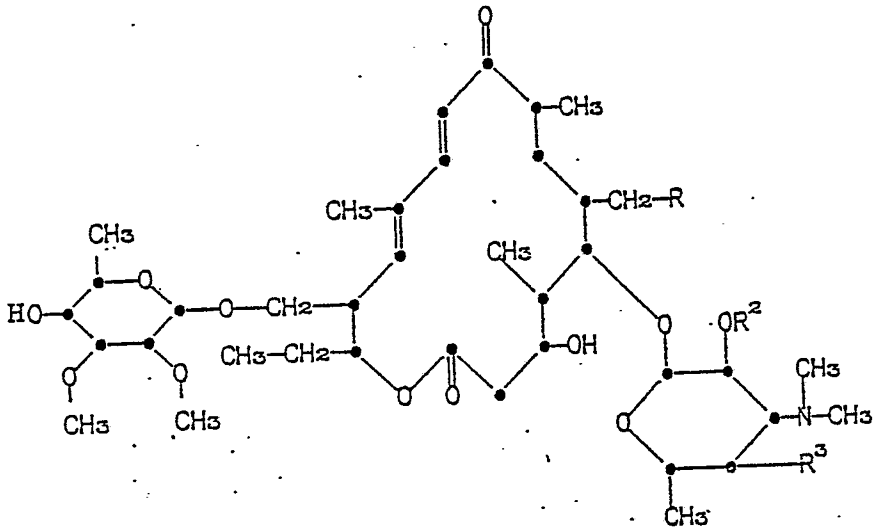

2. A process according to claim 1 wherein R is $CH_2Z$.
3. A process according to claim 2 wherein Z is $OR^4$.
4. A process according to claim 2 wherein Z is $NR^6R^7$.
5. A process according to claim 3 wherein $R^4$ is phenyl benzyl, phenethyl or phenoxyethyl, each of which may have an $R^{11}$ substituent on the ring.
6. A process according to claim 4 wherein $R^6$ and $R^7$ together with the adjacent nitrogen atom form a specified monocyclic ring which may be substituted by $C_1$-$C_4$-alkyl, $C_1$-$C_4$-alkoxy, $C_1$-$C_4$-alkoxycarbonyl, hydroxy, $C_1$-$C_4$-alkanoyloxy, halo, $NR^8R^9$, phenyl or $R^{11}$-substituted phenyl.
7. A process according to claim 6 wherein the monocyclic ring is piperidinyl or substituted piperidinyl.